# EUROPEAN PATENT SPECIFICATION

(11) **EP 1 729 732 B1**
(45) Date of publication and mention of the grant of the patent: **15.04.2009**
(21) Application number: 05718991.2
(22) Date of filing: 16.02.2005
(51) Int. Cl.: A61K 9/16, A61K 47/34, A61K 31/205, A61K 9/70

(54) **ANIONIC HYDROGEL MATRICES WITH PH DEPENDENT MODIFIED RELEASE AS DRUG CARRIERS**
ANIONISCHE HYDROGELMATRIZES MIT PH-ABHÄNGIGER MODIFIZERTER WIRKSTOFFFREISETZUNG ALS ARZNEIMITTELTRÄGER
MATRICES D'HYDROGEL ANIONIQUE À LIBÉRATION MODIFIÉE DEPENDANT DU PH COMME VECTEURS DE MEDICAMENTS

(30) Priority: 01.04.2004 IT RM20040168
(43) Date of publication of application: 13.12.2006
(73) Proprietor: SIGMA-TAU Industrie Farmaceutiche Riunite S.p.A., 00144 Roma (IT)
(72) Inventor: GIAMMONA, Gaetano, c/o Facoltà di Farmacia, I-90123 Palermo (IT); MANDRACCHIA, Delia, c/o Facoltà di Farmacia, I-90123 Palermo (IT)
(74) Representative: Spadaro, Marco
(86) International application number: PCT/IT2005/000081
(87) International publication number: WO 2005/094792

(56) References cited:
- WO-A-00/45792
- US-A- 4 071 508
- US-A1- 2003 152 623
- US-B1- 6 458 386
- PITARRESI G ET AL: "Drug release from alpha,beta-poly(N-2-hydroxyethyl)-DL-aspar tamide-based microparticles" BIOMATERIALS, vol. 25, no. 18, August 2004 (2004-08), pages 4333-4343, XP004497095 ISSN: 0142-9612
- MANDRACCHIA DELIA ET AL: "PH-Sensitive hydrogel based on a novel photocross-linkable copolymer" BIOMACROMOLECULES, vol. 5, no. 5, September 2004 (2004-09), pages 1973-1982, XP002331189 ISSN: 1525-7797 cited in the application
- GIAMMONA G ET AL: "Glycidyl methacrylate derivatization of alpha,beta-poly(N-hydroxyethy l)-dl-aspartamide and alpha,beta-polyasparthydrazide" POLYMER, ELSEVIER SCIENCE PUBLISHERS B.V, GB, vol. 38, no. 13, June 1997 (1997-06), pages 3315-3323, XP004064983 ISSN: 0032-3861
- GIAMMONA G ET AL: "New biodegradable hydrogels based on a photocrosslinkable modified polyaspartamide: synthesis and characterization" BBA - GENERAL SUBJECTS, ELSEVIER SCIENCE PUBLISHERS, NL, vol. 1428, no. 1, 28 June 1999 (1999-06-28), pages 29-38, XP004276327 ISSN: 0304-4165

## Description

The present invention relates to compositions with pH-dependent modified release, consisting of hydrogel matrices containing one or more active ingredients incorporated in them.

The matrices according to the present invention are suitable for releasing said active ingredients in a prolonged pH-dependent manner in given sites of the body.

In the pharmaceutical technology sector it is known that a number of drugs are inserted in formulations modified in such a way as to release said drugs in specific sites of the body.

Thanks to this procedure it is possible: (a) to obtain a prolonged period of action of the drug in the place where it is useful; (b) to avoid instances of rapid release of the drug with side effects at local level, e.g. gastric; (c) to avoid high peak blood levels of the drug, which are the cause of unwanted toxic and side effects at the level of the other districts of the body.

Matrices useful for the delivery of active ingredients are already known in the medical field.

For example., Drug Dev. Ind. Pharm. 13 (6), 1001-1022, (1987) describes a procedure for producing and using colloidal silicate matrices that carry active ingredients.

US 4,608,248 describes a methylcellulose- and hydroxy-methylcellulose-based inert matrix.

EP 0453001 describes a pH-dependent multiparticulate matrix for the controlled release of active ingredients in the lower parts of the intestine..

Additional controlled- or modified-release matrices are also known. In fact, the preparation of modified- or controlled-release matrices can be done using various known techniques, for example, by means of the use of inert matrices in which the main component of the structure of the matrix exerts resistance to the penetration of the solvent due to the weak affinity for aqueous fluids (lipophilia); or by means of the use of bioerodable matrices that are degraded by enzymes in given biological compartments.

The above-mentioned matrices are not without disadvantages; in fact, the inert matrices generally effect an exponential-type uncontrolled release when they enter into contact with body fluids.

While the bioerodable matrices may be ideal in terms of their so-called "controlled-release site", they present the disadvantage of needing an appropriate enzyme or degradation reagent, and in addition, these matrices release metabolites *in situ* that are not completely inert from the toxicological point of view.

In the medical field there is still a strongly perceived need for new modified- or retarded-release matrices, that are biocompatible and of the "controlled-release-site" type.

It has now been found that a new class of pH-dependent controlled-release-site hydrogel matrices are suitable for delivering active ingredients to the ileum and colon, far from the acid milieu of the stomach and for releasing said active ingredients in a prolonged manner.

The hydrogel matrices according to the present invention are obtained in different forms and sizes by chemical reticulation by means of irradiation of copolymers containing photoreticulable groups, in the presence of acid comonomers.

In particular, the anionic hyrogels according to the present invention are obtained starting from derivatised polyaspartamides (PHG and PHM).

The copolymer PHG is obtained by suitably derivatising α,β poly(N-2-hydroxyethyl) D,L aspartamide (PHEA) with glycidyl methacrylate (GMA).

The copolymer PHM is obtained by suitably derivatising α,β poly(N-2-hdroxyethyl) D,L aspartamide (PHEA) with methacrylic anhydride (MA).

PHEA is a polymer with a protein-like structure obtained by reacting a polysuccinimide (PSI) with ethanolamine. This polymer possesses biological and physico-chemical properties such as to make it an excellent candidate for biomedical and pharmaceutical applications (Pitarresi et al., J. Bioact. Compat. Polym. 11; 1996, 328-340; Giammona et al., J. Pharm. Pharmacol. 49; 1997, 1051-1056).

The matrices according to the present invention are not toxic, because they are derived from completely biocompatible substrates..

Said matrices are obtained, for example, by chemical reticulation by means of irradiation of a polyaspartamide derivatised with glycidyl methacrylate or with methacrylic anhydride, in the presence of methacrylic acid. The reticulation leads to the formation of hydrophilic three-dimensional structures insoluble in water, defined as hydrogels. Precisely by virtue of the hydrophilia, these systems absorb water and swell in aqueous media, and the presence of acid groups within the network endows them with pH-sensitive behaviour.

The matrices thus obtained are useful for the delivery and pH-dependent controlled release of active ingredients which are useful in the medical and veterinary fields.

The matrices according to the present invention may be of different forms and sizes, such as, for example, nanoparticles, microparticles, gels, films, cylinders or sponges. The preferred form consists in microparticles..

The drug is incorporated in the matrices before or after the irradiation phase, and subsequent drying.

The object of the present invention therefore consists in anionic hydrogel matrices obtained by chemical reticulation by means of irradiation of a polymer suitably derivatised with photoreticulable groups, in the presence of acid comonomers;
in which the polymer is selected from the group consisting of: polyaminoacid polymers, polyaspartamide polymers, acrylic or methacrylic acid polymers, alkylvinyl polymers, hydroxyalkyl cellulose, carboxyalkyl cellulose, polysaccharides, dextrins, pectins, amides and derivatives, synthetic or natural rubbers or alginic acid; the preferred polymer is α,β poly(N-2-hydroxyethyl)D,L aspartamide (PHEA);
in which said photoreticulable groups are derived from the insertion of glycidyl methacrylate (GMA) or methacrylic anhydride (MA) in the side chain of PHEA;
in which said acid comonomer is selected from methacrylic acid or acrylic acid;
in which said radiation agents are selected from the group consisting of gamma rays, beta rays and ultraviolet rays.

A further object of the present invention consists in anionic hydrogel matrices obtained by chemical reticulation by means of irradiation of a polyaspartamide derivatised with glycidyl methacrylate and with methacrylic anhydride, in the presence of methacrylic or acrylic acid, in which the polyaspartamide is α,β poly(N-2-hydroxyethyl)D,L aspartamide.

A further object of the present invention consists in pharmaceutical compositions for the prolonged pH-controlled release, in the terminal part of the intestine, in the ileum and colon, of one or more active ingredients selected from the group consisting of:
- analgesic agents, such as acetaminophen, phenacetin and sodium salicylate;
- antitussive agents, such as dextromethorphan and codeine phosphate;
- bronchodilators, such as albuterol and procaterol;
- antipsychotics, such as haloperidol and chlorpromazine;
- antihypertensive agents and coronary dilators, such as mono- and dinitrate isosorbide and captopril;
- selective 6-2 antagonists, such as salbutamol, terbutaline, ephedrine, and orciprenaline sulphate;
- calcium antagonists, such as nifedipine, nicardipine, diltiazem and verapamil;
- antiparkinson drugs, such as pergolide, carpidopa and levodopa;
- hormones:

- non-steroidal and steroidal anti-inflammatory drugs, such as ketoprofene, ibuprofene, diclofenac, diflunisal, piroxicam, naproxene, ketorolac, nimesulide, budesonide, tiaprofenic acid, mesalazine (5-aminosalicylic acid), cortisone, hydrocortisone, betamethasone and prednisone;
- antihistamines, such as terfenadine and loratadine;
- antidiarrhoeal and intestinal anti-inflammatory agents, such as loperamide, 5-aminosalicylic acid, olsalazine, sulfasalazine and budenoside;
- spasmolytics, such as octylonium bromide;
- anxiolytics, such as chlordiazepoxides, oxazepam, medazepam, alprazolam, donazepam and lorazepan;
- oral antidiabetic agents, such as glipizide, methformin, phenphormin, gliclazide and glibenclamide;
- cathartics, such as bisacodil and sodium picosulphate;
- antiepileptic agents, such as valproate, carbamazepine, phenytoin and gabapentin;
- anticancer agents;
- disinfectants of the oral cavity or antimicrobials, such as benzalkonium chloride, cetylpyridinium chloride or tibezonium iodide, and a number of aminoderivatives such as benzidamine and chlorhexidine as well as their salts and derivatives;
- sodium fluoride;
- cardioactive agents;
- L-carnitine and/or one or more alkanoyl L-carnitines, in which the straight or branched alkanoyl has 2-6 carbon atoms, such as, for example, acetyl, propionyl, valeryl, isovaleryl, butyryl L-carnitine, or one of their pharmaceutically acceptable salts;
   and possibly one or more conventional excipients, such as, for example, bioadhesives, chitosans, polyacrylamides, natural or synthetic rubbers, and acrylic acid polymers.

What is meant by a pharmaceutically acceptable salt of L-carnitine or of alkanoyl L-carnitines is any salt thereof that does not give rise to toxic or side effects.

These acids are well known to pharmacologists and to experts in pharmacy: Non-limiting examples of these salts are: chloride, bromide, orotate, aspartate, acid aspartate, acid citrate, magnesium citrate, phosphate, acid phosphate, fumarate and acid fumarate, magnesium fumarato, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethane sulphonate, magnesium 2-amino-ethane sulphonate, methane sulphonate, choline tartrate, trichloroacetate, and trifluoroacetate.

What is meant by pharmaceutically acceptable salt of L-carnitine is additionally any salt approved by the FDA and reported in the publication Int. J of Pharm. 33 (1986), 201-217 incorporated herein as a reference.

Preferred are drugs useful for the treatment of chronic intestinal inflammatory diseases, and particularly preferred is propionyl L-carnitine. A further object of the present invention is the use of matrices to deliver and release one or more active ingredients in a prolonged pH-dependent manner, for the preparation of a medicine for the treatment of cardiovascular diseases, tumours, diseases of the central and peripheral nervous systems, and intestinal diseases. Particularly preferred are chronic inflammatory intestinal diseases, such as, for example, chronic ulcerative colitis and Crohn's disease.

The matrices according to the present invention, in the form of cylinders or sponges, lend themselves to being used for the administration of drugs via parenteral or vaginal routes, for example, for the administration of hormones capable of synchronising births in the veterinary field.

The preparation procedure for the anionic hyrogels according to the present invention was carried out using economic methods in terms of both production costs and preparation times.

In this connection, the decision to use radiation, such as, for example, in the form of gamma rays, beta rays and ultraviolet rays, proved extremely advantageous compared to other reticulation methods involving the use of reticulating agents and radical initiators.

In fact, the hydrogels according to the present invention are obtained starting from an aqueous solution of the polyaminoacid derivatives PHG and PHM in the presence of an acid comonomer (acrylic acid or methacrylic acid) using ultraviolet rays, without the addition of any reagent, whereas similar matrices are prepared by means of a reverse-phase suspension polymerisation process, in which the reticulation of the PHG in the presence of acid comonomers (acrylic acid or methacrylic acid) occurs by the addition of reaction initiators and regulators such as ammonium persulphate and TEMED [Muzzalupo R. et al., Colloid Polym. Sci (2001) 279:688-695].

It is clear to any expert in the sector that the use of irradiation proves extremely advantageous in terms of the sterility and subsequent purification of the product, which does not require any particular procedures.

The following examples illustrate the invention.

The synthesis of the anionic hydrogels is done starting from the copolymer PHG, obtained by derivatisation of α,β poly(N-2-hydroxyethyl)D,L aspartamide (PHEA) with glycidyl methacrylate (Giammona et al., Polymer 38 (1997) 3315-3323) and from the copolymer PHM obtained by derivatisation of α,β poly(N-2-hydroxyethyl)D,L aspartamide (PHEA) with methacrylic anhydride [Mandracchia *et al*., Biomacromolecules 5 (2004)]

PHEA, in turn, is derived from the reaction of a polysuccinimide (PSI), prepared by thermal polycondensation of D,L aspartic acid, with ethanolamine in a DMF solution [Neri et. al, J. Med. Chem. 16 (1973) 893; Giammona et al., J. Polym. Sci. Polym. Chem. 25 (1987) 2813].

For the synthesis of the anionic hydrogels according to the present invention a PHEA was used with a weighted average molecular weight of 1,000-100,000, determined by SEC measurements [Mendichi R. et. al., Polymer (2000) 41: 8649-8657].

To obtain PHG, PHEA was derivatised by reaction with glycidyl methacrylate in a solution of anhydrous DMA (dimethylacetamide), using 4-DMAP (4-dimethylaminopyridine) as the catalyst. The reaction was run using different concentrations of catalyst and GMA (glycidl methacrylate), so as to obtain photoreticulable copolymers with different grades of derivatisation. In particular, the following conditions were adopted:
a) A molar ratio of derivatising agent (GMA) to PHEA (repetitive units) ranging from 0.01:1 to 10:1 was used; the preferred ratio ranges from 0.1:1 to 3:1; and particularly preferred is a ratio of 1:1;
b) Molar ratio of the catalyst (DMAP) to the derivatising agent (GMA) in the 0.01:1 to 10:1 range; the preferred ratio is in the 0.1:1 to 3:1 range, and particularly preferred is the ratio of 1.5:1;
c) Temperature: the reaction was run at a constant temperature value ranging from 0 to 60°C; the preferred temperature is 10 to 30°C; and particularly preferred is 25°C;
d) Reaction time: the reaction was run for a period ranging from 1 hour to 10 days; the preferred period is 4 hours to 5 days, and particularly preferred is 48 hours;
   At the end of the reaction, the product obtained was recovered by precipitation in 1-butanol and centrifuged. Various washings were performed with acetone, and the product was dried in vacuo.
   The PHG yield was close to 100% w/w relative to the starting PHEA.
   Each product obtained using the method described was characterised by means of spectrophotometric techniques.
   Solutions of the copolymer PHG thus obtained (1-1000 mg/mL, in a volume of 0.02-25 mL in bidistilled water were placed in pyrex test tubes. To these solutions was added methacrylic or acrylic acid (MAAc or AAc) in a weight-to-weight ratio relative to the PHG ranging from 1 to 80%, the preferred ratio being 10 to 60%, and particularly preferred 40%.
   The test tubes used were equipped with a small internal piston, also made of pyrex, so as to obtain an approximately 2-mm thickness of the solution to be irradiated.
   The solutions, after degassing and insufflation with argon, were subjected to UV irradiation (wavelength from 254 to 366 nm) for a time period ranging from 0.1 to 24 hours.
   The gel thus obtained was purified by effecting a number of washings with distilled water, centrifuging after each washing.
   To obtain PHM, PHEA was derivatised by reaction with methacrylic anhydride (MA) in a solution of anhydrous DMA (dimethylacetamide), using TEA (triethylamine) as the catalyst. The reaction was run using various different concentrations of catalysts and methacrylic anhydride, so as to obtain photoreticulable copolymers with different derivatisation grades. In particular the following conditions were adopted:
e) A molar ratio of derivatising agent (MA) to PHEA (repetitive units) ranging from 0.01:1 to 10:1 was used; the preferred ratio ranges from 0.1: to 1:1; and particularly preferred is a ratio of 0.5:1;
f) Molar ratio of the catalyst (TEA) to the derivatising agent (MA) in the 0.01:1 to 10:1 range; the preferred ratio is in the 0.1:1 to 1:1 range, and particularly preferred is the ratio of 0.5:1;
g) Temperature: the reaction was run at a constant temperature value ranging from 0 to 80°C; the preferred temperature is 10 to 60°C; and particularly preferred is 40°C;
h) Reaction time: the reaction was run for a period ranging from 1 hour to 10 days; the preferred period is 4 hours to 5 days, and particularly preferred is 48 hours;
   At the end of the reaction, the product obtained was recovered by precipitation in 2-propanol and centrifuged. Various washings were performed with 2-propanol and acetone, and the product was dried in vacuo.

The PHM yield was close to 100% w/w relative to the starting PHEA.

Each product obtained using the method described was characterised by means of spectrophotometric techniques.

Solutions of the copolymer PHM thus obtained (1-1000 mg/mL, in a volume of 0.02-25 mL in bidistilled water were placed in pyrex test tubes. To these solutions was added methacrylic or acrylic acid (MAAc or AAc) in a weight-to- weight ratio in relation to the PHM ranging from 1 to 80%, the preferred ratio being 10 to 60%, and particularly preferred 20%.

The test tubes used were equipped with a small internal piston, also made of pyrex, so as to obtain an approximately 2-mm thickness of the solution to be irradiated.

The solutions, after degassing and insufflation with argon, were subjected to UV irradiation (wavelength from 254 to 366 nm) for a time period ranging from 0.1 to 24 hours.

The gel thus obtained was purified by effecting a number of washings with distilled water, centrifuging after each washing.

These pH-sensitive matrices are loaded with active ingredients by means of two main procedures. One procedure consists in the incorporation of the drug.during the preparation of the hydrogel and, then, during the irradiation phase; the other, by contrast, consists in loading the drug by impregnating the previously prepared matrix.

The hydrogels obtained were characterised by means of spectrophotometric techniques and swelling studies in distilled water and in media simulating a number of body fluids (gastric juice, intestinal fluid), temperature range 0° to 60°C). The swelling values indicated a strong affinity of the hydrogels prepared according to the present invention for an aqueous medium, the extent of which proved to be dependent upon the reticulation grade of the product and on the pH and composition of the swelling medium (pH range analysed: from 1 to 9).

### Example 1

### Synthesis of copolymer PHG-based anionic hydrogels reticulated by ultraviolet irradiation

To a solution of PHEA in anhydrous DMA (500 mg/10 mL) were added 579 mg of 4-dimethylaminopyridine (4-DMAP) as the catalyst. To this solution were added 420 microlitres of glycidyl methacrylate (GMA) to obtain the copolymer PHG containing vinyl groups.

The above-mentioned amounts are in agreement with the following ratios:

Molar ratio of derivatising agent (GMA) to PHEA (repetitive units) = 1:1.

Molar ratio of catalyst (DMAP) to derivatising agent (GMA) = 1.5:1

The reaction was run at a constant temperature value of 25°C and for a time period of 48 hours.

On completion of the reaction time, the product was recovered by precipitation in 1-butanol and centrifuged at 12000 rpm for 10 minutes at 4°C.

Various washings were done with acetone, and the product was dried in vacuo.

The PHG yield was 98 ± 1% w/w in relation the starting PHEA.

To an aqueous solution of the copolymer PHG thus obtained (60 mg/mL) was added methacrylic acid (MAAc) in an amount equal to 40% w/w in relation to the starting PHG. This solution, after being deprived of the dissolved oxygen, was subjected to ultraviolet irradiation at a wavelength of 313 nm for a time period of 3.5 hours.

The hydrogel was recovered, purified by a number of washings with distilled water and dried by means of liophilisation. The product obtained was weighed (yield: 97% w/w) and characterised by means of spectrophotometric techniques.

In swelling studies at different pH values the product displayed strong affinity for aqueous media and a pH-sensitive behaviour pattern.

### Example 2

### Synthesis of copolymer PHM-based anionic hydrogels reticulated by means of ultraviolet irradiation

To a solution of PHEA in anhydrous DMA (500 mg/10 mL) were added 79.44 mg of triethylamine (TEA) as the catalyst. 242 mg of methacrylic anhydride (MA were added to this solution in order to obtain the copolymer PHM containing vinyl groups.

The above-mentioned amounts are in agreement with the following ratios:

Molar ratio of derivatising agent (MA) to PHEA (repetitive units) = 0.5:1

Molar ratio of catalyst (TEA) to derivatising agent (MA) = 0.5:1

The reaction was run at a constant temperature value of 40°C and for a time period of 48 hours.

On completion of the reaction time, the product was recovered by precipitation in 2-propanol and centrifuged at 12000 rpm for 10 minutes at 4°C.

Various washings were done with 2-propanol and acetone, and the product was dried in vacuo.

The PHM yield was 98 ± 1% w/w relative to the starting PHEA.

To an aqueous solution of the copolymer PHM thus obtained (60 mg/mL) was added methacrylic acid (MAAc) in an amount equal to 20% w/w relative to the starting PHM. This solution, after being deprived of the dissolved oxygen, was subjected to ultraviolet irradiation at a wavelength of 313 nm for a time period of 3.5 hours.

The hydrogel was recovered, purified by a number of washings with distilled water and dried by means of liophilisation. The product obtained was weighed (yield: 97% w/w) and characterised by means of spectrophotometric techniques.

In swelling studies at different pH values the product displayed strong affinity for aqueous media and a pH-sensitive behaviour pattern.

### Example 3

### Incorporation of Propionyl L-carnitine hydrochloride in the PHG-MAAc matrix

Propionyl L-carnitine hyrochloride (PLC) was loaded into the gel during the reticulation phase.

In particular, the hydrogel containing PLC was prepared by UV irradiation of a solution of the copolymer PHG (60 mg/mL), MAAc (40% by weight relative to PHG) and PLC (50 mg/mL) in bidistilled water.

Irradiation with UV rays was done for 3.5 hours under argon at 313 nm.

After the irradiation, the sample was recovered and liophilised.

pH-related controlled release tests showed that an aliquot of PLC (approximately 30%) is released in solutions at pH 1 in a time period of approximately 2 ore (the PLC component released is that located on the surface of the matrix or in the surface layers thereof). On modifying the pH of the same solution there is a further release of approximately 40% of the PLC (total released: 70%) within a further 4 hours.

In *in*-*vivo* conditions the remaining 30% of the PLC will be released during the complete degradation of the matrix in the terminal parts of the large bowel during the following hours in which the matrix remains in the digestive tract.

### Example 4

### Incoporation of propionyl L-carnitine hydrochloride in the PHM-MAAc matrix

Propionyl L-carnitine hydrochloride (PLC) was loaded into the gel during the reticulation phase.

In particular, the hydrogel containing PLC was prepared by means of UV irradiation of a solution of the copolymer PHM (60 mg/mL), MAAc (20% by weight relative to PHM) and PLC (50 mg/mL) in bidistilled water.

Irradiation with UV rays was done for 3.5 hours under argon at 313 nm.

After the irradiation, the sample was recovered and liophilised.

pH-related controlled release tests showed that an aliquot of PLC (approximately 60%) is released in solutions at pH 1 in a time period of approximately 2 hours (the PLC component released is that located on the surface of the matrix or in the surface layers thereof). On modifying the pH of the same solution there is a further release of approximately 20% of the PLC (total released: 80%) within a further 4 hours.

In in-vivo conditions the remaining 20% of the PLC will be released during the complete degradation of the matrix in the terminal parts of the large bowel during the following hours in which the matrix remains in the digestive tract.

## Claims

1. Anionic hydrogel matrix obtained by chemical reticulation by means of irradiation of a polymer derivatised with photoreticulable groups which are derived from the insertion of glycidyl methacrylate (GMA) or methacrylic anhydride (MA) in the side chain, in the presence of acid comonomers.

2. Matrix according to claim 1, in which the polymer is a polyaminoacid or a polyaspartamide polymer.

3. Matrix according to claim 1 or 2, in which the polymer is α,β poly (N-2-hydroxyethyl) D, L aspartamide (PHEA).

4. Matrix according to claim 1, in which the acid comonomer is selected from methacrylic acid or acrylic acid.

5. Matrix according to claim 1, in which the irradiation agents are selected from the group consisting of gamma rays, beta rays and ultraviolet radiation.

6. Matrix according to claim 1, in the form of nanoparticles, microparticles, gels; films, cylinders or sponges, the preferred form being microparticles.

7. Composition consisting of the matrix of claim 1, and one or more active ingredients.

8. Composition according to claim 7, further comprising one or more pharmaceutically acceptable excipients.

9. Composition according to claim 8, in which the excipients are selected from the group consisting of bioadhesives, chitosans, polyacrylamides, natural or synthetic rubbers and acrylic acid polymers.

10. Composition according to claim 7, in which said active ingredients are selected from the group consisting of: - analgesic agents, such as acetaminophen,phenacetin and sodium salicylate; -antitussive agents, such as dextromethorphan and codeine phosphate; - bronchodilators, such as albuterol and procaterol; - antipsychotics, such ashaloperidol and chlorpromazine; - antihypertensive agents and coronary dilators, such as mono-and dinitrate isosorbide and captopril; -selective 6-2 antagonists, such as salbutamol, terbutaline, ephedrine, andorciprenaline sulphate; calcium antagonists, such as nifedipine, nicardipine, iltiazem and verapamil; - antiparkinson drugs, such as pergolide, carpidopa and levodopa; - hormones ; non-steroidal and steroidal anti-inflammatory drugs, such as ketoprofene, ibuprofene, diclofenac, diflunisal, piroxicam, naproxene, ketorolac, nimesulide, budesonide, tiaprofenic acid, mesalazine (5- aminosalicylic acid), cortisone, hydrocortisone, betamethasone and prednisone; - antihistamines, such as terfenadine and loratadine;-antidiarrhoeal and intestinal anti-inflammatory agents, such as loperamide, 5-aminosalicylic acid, olsalazine, sulfasalazine and budenoside;- spasmolytics, such asoctylonium bromide;- anxiolytics, such as chlordiazepoxides, oxazepam, medazepam,alprazolam, donazepam and lorazepan;- oral antidiabetic agents, such as glipizide, methformin, phenphormin, gliclazide and glibenclamide; - cathartics, such as bisacodil and sodium picosulphate; - antiepileptic agents, such asvalproate, carbamazepine, phenytoin and gabapentin; - anticancer agents; - disinfectants of the oral cavity or antimicrobials, such as benzalkonium chloride, cetylpyridinium chloride or tibezonium iodide, and a number of aminoderivatives such as benzidamine and chlorhexidine as well as their salts and derivatives; - sodium fluoride; - cardioactive agents; - antihistamines; L-carnitine and/or one or more alkanoyl L-carnitines, or one of their pharmaceutically acceptable salts.

11. Composition according to claim 10, in which the alkanoyl, straight or branched, has 2-6 carbon atoms, and is selected from the group consisting of acetyl, propionyl, butyryl, valeryl or isovaleryl L- carnitine.

12. Composition according to claim 10, in which said pharmaceutically acceptable salt of L-carnitine or of the alkanoyl L-carnitines is selected from the group consisting of chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethane sulphonate, magnesium 2-amino-ethane sulphonate, methane sulphonate, choline tartrate, trichloroacetate, and trifluoroacetate.

13. Composition according to claims 7-12 for oral or parenteral use.

14. The composition of claim 7 for use in human and veterinary medicine.

15. The composition of claim 7, for use in the treatment of cardiovascular diseases, tumours, central and peripheral nervous system diseases, or intestinal diseases.

16. Use of the composition of claim 7, for the preparation of a medicine for the treatment of cardiovascular diseases, tumours, central and peripheral nervous system diseases, or intestinal diseases.

17. Use according to claim 15 or 16, in which the intestinal disease is chronic ulcerative colitis or Crohn's disease.

18. Use according to claim 17, in which the drug useful for the treatment of chronic intestinal disease is propionyl L-carnitine.

19. Use according to claim 14, in which said composition can be administered by oral, parenteral or vaginal routes.

## Patentansprüche

1. Anionische Hydrogelmatrix, erhalten durch chemische Vernetzung mittels Bestrahlung eines Polymers, das mit photovernetzbaren Gruppen derivatisiert ist, die hergeleitet werden aus der Einführung von Glycidylmethacrylat (GMA) oder Methacrylsäureanhydrid (MA) in die Seitenkette, in Anwesenheit von Säure-Comonomeren.

2. Matrix gemäß Anspruch 1, in welcher das Polymer eine Polyaminosäure oder ein Polyaspartamidpolymer ist.

3. Matrix gemäß Anspruch 1 oder 2, in welcher das Polymer ein α,β-Poly-(N-2-hydroxyethyl)-D,L-aspartamid (PHEA) ist.

4. Matrix gemäß Anspruch 1, in welcher das Säure-Comonomer ausgewählt ist aus Methacrylsäure oder Acrylsäure.

5. Matrix gemäß Anspruch 1, in welcher die Bestrahlungsmittel ausgewählt sind aus der Gruppe, die aus Gammastrahlen, Betastrahlen und ultravioletter Strahlung besteht.

6. Matrix gemäß Anspruch 1, in der Form von Nanopartikeln, Mikropartikeln, Gelen, Filmen, Zylindern oder Schwämmen, wobei die bevorzugte Form Mikropartikel sind.

7. Zusammensetzung bestehend aus der Matrix nach Anspruch 1 und einem aktiven Bestandteil oder mehreren aktiven Bestandteilen.

8. Zusammensetzung gemäß Anspruch 7, weiterhin einen pharmazeutisch verträglichen Hilfsstoff oder mehrere pharmazeutisch verträgliche Hilfsstoffe umfassend.

9. Zusammensetzung gemäß Anspruch 8, in welcher die Hilfsstoffe ausgewählt sind aus der Gruppe, die aus Bioadhäsiva, Chitosanen, Polyacrylamiden, natürlichen oder synthetischen Gummis und Acrylsäurepolymeren besteht.

10. Zusammensetzung gemäß Anspruch 7, in welcher die genannten aktiven Bestandteile ausgewählt sind aus der Gruppe, bestehend aus: - analgetischen Agenzien, wie Acetaminophen, Phenacetin und Natriumsalicylat; - antitussiven Agenzien, wie Dextromethorphan und Codeinphosphat; - Bronchodilatatoren, wie Albuterol und Procaterol; - Antipsychotika, wie Ashaloperidol und Chlorpromazin; - antihypertensiven Mitteln und Koronardilatatoren, wie Isosorbidmono- und -dinitrat und Captopril; - selektiven 6-2-Antagonisten, wie Salbutamol, Terbutalin, Ephedrin, Andorciprenalinsulfat; - Calciumantagonisten, wie Nifedipin, Nicardipin, 11-tiazem und Verapamil; - Antiparkinson-Arzneimitteln, wie Pergolid, Carpidopa und Levodopa; - Hormonen; nicht-steroidalen und steroidalen entzündungshemmenden Arzneimitteln, wie Ketoprofen, Ibuprofen, Diclofenac, Diflunisal, Piroxicam, Naproxen, Ketorolac, Nimesulid, Budesonid, Tiaprofensäure, Mesalazin (5-Aminosalicylsäure), Cortison, Hydrocortison, Betamethason und Prednison; - Antihistaminen, wie Terfenadin und Loratadin; - Antidiarrhoika und Darmentzündung hemmenden Mitteln, wie Loperamid, 5-Aminosalicylsäure, Olsalazin, Sulfasalazin und Budenosid; - Spasmolytika, wie Asoctyloniumbromid; - Anxiolytika, wie Chlordiazepoxiden, Oxazepam, Medazepam, Alprazolam, Donazepam und Lorazepan; - oralen Antidiebatika, wie Glipizid, Methformin, Phenphormin, Gliclazid und Glibenclamid; - Kathartika, wie Bisacodil und Natriumpicosulfat; - Antiepileptika, wie Asvalproat, Carbamazepin, Phenytoin und Gabapentin; - Antikrebsmitteln; - Desinfektionsmitteln der Mundhöhle oder antimikrobiellen Mitteln, wie Benzalkoniumchlorid, Cetylpyridiniumchlorid oder Tibezoniumiodid, und einer Anzahl von Aminoderivaten wie Benzidamin und Chlorhexidin, wie auch ihren Salzen und Derivaten; - Natriumfluorid; - kardioaktiven Mitteln; - Antihistaminen; L-Carnitin und/oder einem Alkanoyl-L-carnitin oder mehreren Alkanoyl-L-carnitinen, oder einem ihrer pharmazeutisch verträglichen Salze.

11. Zusammensetzung gemäß Anspruch 10, in welcher das Alkanoyl, gerade oder verzweigt, 2-6 Kohlenstoffatome besitzt und ausgewählt ist aus der Gruppe, die aus Acetyl-, Propionyl-, Butyryl-, Valeryl- oder Isovaleryl-L-carnitin besteht.

12. Zusammensetzung gemäß Anspruch 10, in welcher das genannte pharmazeutisch verträgliche Salz von L-Carnitin oder der Alkanoyl-L-carnitine ausgewählt ist aus der Gruppe, bestehend aus Chlorid, Bromid, Orotat, Aspartat, saurem Aspartat, saurem Citrat, Magnesiumcitrat, Phosphat, saurem Phosphat, Fumarat und saurem Fumarat, Magnesiumfumarat, Lactat, Maleat und saurem Maleat, Oxalat, saurem Oxalat, Pamoat, saurem Pamoat, Sulfat, saurem Sulfat, Glucosephosphat, Tartrat und saurem Tartrat, Glycerophosphat, Mucat, Magnesiumtartrat, 2-Amino-ethansulfonat, Magnesium-2-amino-ethansulfonat, Methansulfonat, Cholintartrat, Trichloracetat und Trifluoracetat.

13. Zusammensetzung gemäß den Ansprüchen 7-12 zur oralen oder parenteralen Verwendung.

14. Zusammensetzung nach Anspruch 7, zur Verwendung in Human- und Veterinärmedizin.

15. Zusammensetzung nach Anspruch 7, zur Verwendung bei der Behandlung von kardiovaskulären Krankheiten, Tumoren, Krankheiten des zentralen oder peripheren Nervensystems oder Darmkrankheiten.

16. Verwendung der Zusammensetzung nach Anspruch 7, zur Herstellung eines Medikaments zur Behandlung von kardiovaskulären Krankheiten, Tumoren, Krankheiten des zentralen oder peripheren Nervensystems oder Darmkrankheiten.

17. Verwendung gemäß Anspruch 15 oder 16, bei welcher die Darmkrankheit chronische Colitis ulcerosa oder Morbus Crohn ist.

18. Verwendung gemäß Anspruch 17, bei welcher das Arzneimittel, das zur Behandlung von chronischer Darmkrankheit geeignet ist, Propionyl-L-carnitin ist.

19. Verwendung gemäß Anspruch 14, bei welcher die genannte Zusammensetzung auf oralen, parenteralen oder vaginalen Wegen verabreicht werden kann.

## Revendications

1. Matrice d'hydrogel anionique obtenue par réticulation chimique au moyen de l'irradiation d'un polymère modifié avec des groupes photoréticulables qui sont obtenus par l'insertion de méthacrylate de glycidyle (GMA) ou d'anhydride méthacrylique (MA) dans la chaîne latérale, en présence de comonomères acides.

2. Matrice selon la revendication 1, dans laquelle le polymère est un polyaminoacide ou un polymère polyaspartamide.

3. Matrice selon la revendication 1 ou 2, dans laquelle le polymère est un α,β poly(N-2-hydroxyéthyl) D,L aspartamide (PHEA).

4. Matrice selon la revendication 1, dans laquelle le comonomère acide est choisi parmi l'acide méthacrylique ou l'acide acrylique.

5. Matrice selon la revendication 1, dans laquelle les agents d'irradiation sont choisis dans le groupe constitué par les rayons gamma, les rayons bêta et une radiation ultraviolette.

6. Matrice selon la revendication 1, sous la forme de nanoparticules, de microparticules, de gels ; de films, de cylindres ou d'éponges, la forme préférée étant les microparticules.

7. Composition consistant en la matrice de la revendication 1 et un ou plusieurs ingrédients actifs.

8. Composition selon la revendication 7, comprenant en outre un ou plusieurs excipients pharmaceutiquement acceptables.

9. Composition selon la revendication 8, dans laquelle les excipients sont choisis dans le groupe constitué par les bioadhésifs, les chitosans, les polyacrylamides, les caoutchoucs naturels ou synthétiques et les polymères de l'acide acrylique.

10. Composition selon la revendication 7, dans laquelle lesdits ingrédients actifs sont choisis dans le groupe constitué par: les agents analgésiques, tels que l'acétaminophène, la phénacétine et le salicylate de sodium ; les agents antitussifs tels que le dextrométhorphane et le phosphate de codéine ; les broncho-dilatateurs tels que l'albutérol et le procatérol; les antipsychotiques tels que l'halopéridol et la chlorpromazine ; les agents antihypertenseurs et les dilatateurs coronaires tels que le mono et dinitrate d'isosorbide et le captopril ; les antagonistes 6-2 sélectifs tels que le salbutamol, la terbutaline, l'éphédrine et le sulfate d'orciprénaline ; les antagonistes du calcium tels que la nifédipine, la nicardipine, l'iltiazem et le Vérapamil; les substances médicamenteuses antiparkinsoniennes telles que le pergolide, le carpidopa et le lévodopa; les hormones ; les substances médicamenteuses anti-inflammatoires non stéroïdiennes et stéroïdiennes telles que le kétoprofène, l'ibuprofène, le diclofénac, le diflunisal, le piroxicam, le naproxène, le kétorolac, le nimésulide, le budésonide, l'acide tiaprofénique, la mésalazine (acide 5-aminosalicylique), la cortisone, l'hydrocortisone, la bétaméthasone et la prednisone; les antihistaminiques tels que la terfénadine et la loratadine ; les agents antidiarrhéiques et anti-inflammatoires intestinaux tels que le lopéramide, l'acide 5-aminosalicylique, l'olsalazine, la sulfasalazine et le budénoside ; les spasmolytiques tels que le bromure d'octylonium ; les anxiolytiques tels que les chlordiazépoxydes, l'oxazépam, le médazépam, l'alprazolam, le donazépam et le lorazépan ; les agents antidiabétiques oraux tels que le glipizide, la metformine, la phenphormine, le glicazide et le glibenclamide ; les cathartiques tels que le bisacodil et le picosulfate de sodium ; les agents antiépileptiques tels que le valproate, la carbamazépine, la phénytoïne et la gabapentine ; les agents anticancéreux ; les désinfectants de la cavité buccale ou les antimicrobiens tels que le chlorure de benzalkonium, le chlorure de cétylpyridinium ou l'iodure de tibézonium, et un certain nombre de dérivés amino tels que la benzidamine et la chlorhexidine ainsi que leurs sels et dérivés; le fluorure de sodium ; les agents cardioactifs ; les antihistaminiques; la L-carnitine et/ou une ou plusieurs alkanoyle L-carnitines, ou l'un de leurs sels pharmaceutiquement acceptables.

11. Composition selon la revendication 10, dans laquelle l'alkanoyle, linéaire ou ramifié, a 2 à 6 atomes de carbone et est choisi dans le groupe constitué par acétyle, proprionyle, butyryle, valéryle ou L-carnitine isovaléryle.

12. Composition selon la revendication 10, dans laquelle ledit sel pharmaceutiquement acceptable de L-carnitine ou des alkanoyle L-carnitines est choisi dans le groupe constitué par un chlorure, un bromure, un orotate, un aspartate, un aspartate d'acide, un citrate d'acide, le citrate de magnésium, un phosphate, un phosphate d'acide, un fumarate et un fumarate d'acide, le fumarate de magnésium, un lactate, un maléate et un maléate d'acide, un oxalate, un oxalate d'acide, un pamoate, un pamoate d'acide, un sulfate, un sulfate d'acide, le phosphate de glucose, un tartrate et un tartrate d'acide, un glycéro-phosphate, un mucate, le tartrate de magnésium, le sulfonate de 2-amino-éthane, le sulfonate de magnésium 2-amino-éthane, le sulfonate de méthane, le tartrate de choline, un trichloroacétate et un trifluoroacétate.

13. Composition selon les revendications 7 à 12 pour l'utilisation orale ou parentérale.

14. Composition selon la revendication 7, pour l'utilisation en médecine humaine et vétérinaire.

15. Composition selon la revendication 7, pour l'utilisation dans le traitement de maladies cardiovasculaires, de tumeurs, de maladies du système nerveux central et périphérique ou de maladies intestinales.

16. Utilisation de la composition selon la revendication 7, pour la préparation d'un médicament pour le traitement de maladies cardiovasculaires, de tumeurs, de maladies du système nerveux central et périphérique ou de maladies intestinales.

17. Utilisation selon la revendication 15 ou 16, dans laquelle la maladie intestinale est une colite ulcérative chronique ou une maladie de Crohn.

18. Utilisation selon la revendication 17, dans laquelle la substance médicamenteuse utile pour le traitement d'une maladie intestinale chronique est la L-carnitine propionyle.

19. Utilisation selon la revendication 14, dans laquelle ladite composition peut être administrée par les voies orale, parentérale ou vaginale.
